# EUROPEAN PATENT APPLICATION

(11) **EP 2 368 482 A1**
(43) Date of publication of application: **28.09.2011**
(21) Application number: 11152570.5
(22) Date of filing: 28.01.2011
(51) Int. Cl.: A61B 1/01

(54) **Handle device and overtube apparatus**

(30) Priority: 25.03.2010 JP 2010070058
(71) Applicant: Fujifilm Corporation, Minato-ku Tokyo (JP)
(72) Inventor: YAMAMOTO, Seiichi, Kanagawa (JP)
(74) Representative: Klunker . Schmitt-Nilson . Hirsch

(57) **Abstract**

A handle device of a barrel shape is combined with a proximal end (60a, 63) of an overtube apparatus (11) for receiving entry of an insertion tube (13) of an endoscope (10) to guide the insertion tube into a body cavity. The handle device has a small diameter portion (76). First and second large diameter portions (77a, 77b) are disposed on sides of distal and proximal ends (78a, 78b) of the small diameter portion, and have a larger diameter than the small diameter portion. A radial difference (dA, dB) between each of the first and second large diameter portions and the small diameter portion is 3.5-8 mm. First and second inclined portions (78a, 78b) are arranged to extend from the small diameter portion to respectively the first and second large diameter portions, and inclined at a rising angle (θA, θB) of 30-80 degrees with reference to the small diameter portion. The handle device is formed from a material having a Shore A hardness of 90 or more.

## Description

### BACKGROUND OF THE INVENTION

### 1. Field of the Invention

The present invention relates to a handle device and overtube apparatus. More particularly, the present invention relates to a handle device which can have a simplified structure for an overtube apparatus, and can prevent slip of fingers holding the overtube apparatus.

### 2. Description Related to the Prior Art

An endoscope is used for medical imaging of a body cavity of a patient, and has an insertion tube or elongated tube. For diagnosis and treatment, the insertion tube is entered in a gastrointestinal tract as deeply as an intestine and colon. The intestine and colon are considerably tortuous. Entry of the insertion tube of the endoscope in the intestine and colon is highly difficult, because force of its manipulation of a doctor or operator is difficult to transmit to a distal end of the insertion tube. There is a known method of using an overtube apparatus or sliding tool. The insertion tube of the endoscope is inserted in a tube lumen of the overtube apparatus, and is entered into a body cavity together with the overtube apparatus. The overtube apparatus can guide the insertion tube of the endoscope, and thus makes it possible to manipulate the insertion tube to reach a deep site in the gastrointestinal tract, because occurrence of an unwanted bend or tortuous form of the insertion tube can be prevented.

To use the overtube apparatus, a coating of lubricating jelly is applied to its tube surface for facilitating entry into the body. However, slip of a hand of a doctor or operator may occur because the lubricating jelly deposits on the hand. A handle device is combined with the overtube apparatus for manual handling. In the field of the overtube apparatus, the handle device of low slipping property and high grippability is preferred. WO 2007/032085 and JP-A 2001-321328 disclose the handle device including a small diameter portion and large diameter portions. The large diameter portions are disposed on distal and proximal sides of the small diameter portion, and have a larger diameter than the small diameter portion. When his or her fingers grasp the small diameter portion, the handle device can be held reliably. Also, the large diameter portions prevent slip because of tight holding of the fingers on the large diameter portions.

However, the large diameter portions are oriented to extend perpendicularly from the small diameter portion in the handle device of WO 2007/032085 and JP-A 2001-321328. Fingers may feel pain upon touch of angular corners of the large diameter portions specifically when fingers exert force to the handle device for a long time or in forward and backward movement of the overtube apparatus.

If silicone resin is used for forming the handle device, load to fingers on the handle device may be reduced. However, the silicone resin is very expensive to raise a manufacturing cost of the handle device seriously.

### SUMMARY OF THE INVENTION

In view of the foregoing problems, an object of the present invention is to provide a handle device which can have a simplified structure for an overtube apparatus, and can prevent slip of fingers holding the overtube apparatus.

In order to achieve the above and other objects and advantages of this invention, a handle device of a barrel shape for use at a proximal end of an overtube apparatus for receiving entry of an insertion tube of an endoscope to guide the insertion tube into a body cavity is provided. There is a small diameter portion. First and second large diameter portions are disposed on sides of distal and proximal ends of the small diameter portion, have a larger diameter than the small diameter portion, wherein a radial difference between each of the first and second large diameter portions and the small diameter portion is equal to or more than 3.5 mm and equal to or less than 8 mm. First and second inclined portions are arranged to extend from the small diameter portion to respectively the first and second large diameter portions, and inclined at a rising angle equal to or more than 30 degrees and equal to or less than 80 degrees with reference to the small diameter portion. The small diameter portion, the first and second large diameter portions, and the first and second inclined portions are formed from a material having a Shore A hardness equal to or more than 90.

The material is constituted by at least one of ABS resin, polypropylene, polyethylene, and polyvinyl chloride.

Furthermore, a fluid channel supplies air for a balloon positioned at a distal end of the overtube apparatus in communication with a lumen in the overtube apparatus.

Furthermore, a handle sleeve has a handle peripheral surface on which the small diameter portion, the first and second large diameter portions and the first and second inclined portions are formed. A handle lumen is defined through the handle sleeve, for receiving entry of the insertion tube.

The overtube apparatus has a proximal end opening of an elongated tube thereof, and a distal end of the handle sleeve is inserted in the proximal end opening for connection.

The first large diameter portion has a smaller diameter than a diameter of the second large diameter portion.

Furthermore, a proximal opening is formed to communicate with a proximal end of the handle lumen, and to extend through the second large diameter portion, the proximal opening having an inner diameter larger than an inner diameter of the handle lumen.

The fluid channel includes a first conduit, positioned on the handle peripheral surface, for extending crosswise to a longitudinal axis of the handle lumen. A second conduit communicates with an end of the first conduit close to the longitudinal axis, and for extending along the longitudinal axis in a distal direction.

The fluid channel is constituted by an end tube portion formed with the handle peripheral surface and on a distal side from the first large diameter portion.

Also, an overtube apparatus for receiving entry of an insertion tube of an endoscope is provided, and includes an elongated tube for entry in a body cavity, the elongated tube having a tube lumen for receiving the insertion tube. A handle device of a barrel shape is connected to a proximal end of the elongated tube. The handle device includes a small diameter portion. First and second large diameter portions are disposed on sides of distal and proximal ends of the small diameter portion, have a larger diameter than the small diameter portion, wherein a radial difference between each of the first and second large diameter portions and the small diameter portion is equal to or more than 3.5 mm and equal to or less than 8 mm. First and second inclined portions are arranged to extend from the small diameter portion to respectively the first and second large diameter portions, and inclined at a rising angle equal to or more than 30 degrees and equal to or less than 80 degrees with reference to the small diameter portion. The small diameter portion, the first and second large diameter portions, and the first and second inclined portions are formed from a material having a Shore A hardness equal to or more than 90.

Also, an endoscope system includes an endoscope having an insertion tube. An overtube apparatus receives entry of the endoscope to guide. The overtube apparatus includes a handle device as defined in the definition heretofore. An elongated tube is adapted for entry in the body cavity, has the proximal end and a tube lumen, the proximal end being provided with the handle device secured thereto, the tube lumen receiving the insertion tube.

Consequently, it is possible to prevent slip of fingers holding the overtube apparatus, because the inclined portions operate for easy handling of fingers of an operator, and because of the use of the material having a sufficiently high hardness.

### BRIEF DESCRIPTION OF THE DRAWINGS

The above objects and advantages of the present invention will become more apparent from the following detailed description when read in connection with the accompanying drawings, in which:
Fig. 1 is an explanatory view in a plan, illustrating an endoscope system;
Fig. 2 is a perspective view illustrating a handle device and an overtube apparatus;
Fig. 3 is a vertical section illustrating the handle device;
Fig. 4 is a perspective view illustrating a use of the overtube apparatus;
Fig. 5 is a perspective view illustrating another preferred embodiment of the use of the overtube apparatus.

### DETAILED DESCRIPTION OF THE PREFERRED EMBODIMENT(S) OF THE PRESENT INVENTION

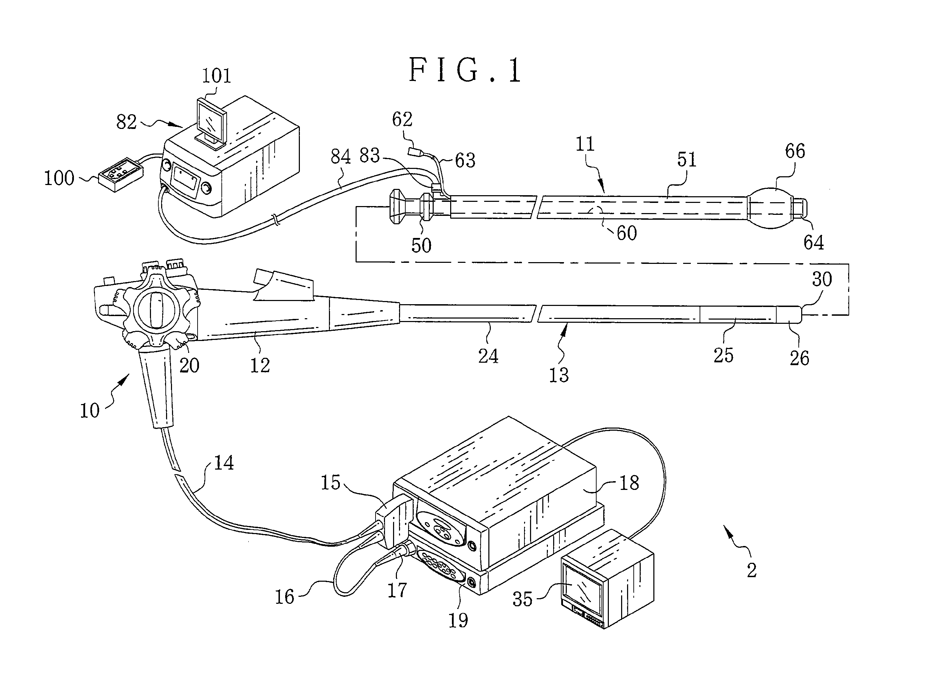

In Fig. 1, an endoscope system 2 includes an electronic endoscope 10 and a balloon tube apparatus or overtube apparatus 11. The endoscope 10 includes a handle 12 and an elongated tube 13 or insertion tube which extends from the handle 12 and is used for entry in a gastrointestinal tract as a body cavity, for example, large intestine. A universal cable 14 is connected to the handle 12. A light source connector 15 or plug is disposed at an end of the universal cable 14. A cable 16 extends from an end of the light source connector 15 as a branch. A connector 17 or plug is disposed at an end of the cable 16. A light source apparatus 18 is connected by use of the light source connector 15. A processing apparatus 19 is connected by use of the connector 17.

The insertion tube 13 includes a flexible unit 24, a steering device 25 and a head assembly 26 in an order of a distal direction on the handle 12. The flexible unit 24 is as long as several meters for reach of the head assembly 26 to an object of interest in a body cavity. In rotational operation of a steering wheel 20 at the handle 12, the steering device 25 is steered with bends in upward and downward directions and directions to the right and left. Thus, it is possible to direct the head assembly 26 in any direction toward an intended point in the body cavity.

In a distal surface 30 of the head assembly 26, an imaging window is formed for receiving image light from an object in a body cavity. An image pickup device (not shown) is incorporated in the head assembly 26 for image pickup of the image light entered through the imaging window, for example, a CCD. The processing apparatus 19 processes an image signal in the image processing of various functions into a video signal after transmission of the image signal through a signal cable from the image pickup device. A monitor display panel 35 is caused to display an image according to the video signal. See Fig. 1.

The overtube apparatus 11 includes a handle device 50 and an elongated tube 51. The handle device 50 of the invention is a plastic part formed from rigid resin such as ABS resin. The elongated tube 51 is formed from flexible resin such as polyurethane foam. A balloon 66 to be described later is disposed at a distal end of the elongated tube 51. A proximal end of the elongated tube 51 is fitted on a periphery of a distal end of the handle device 50 fixedly.

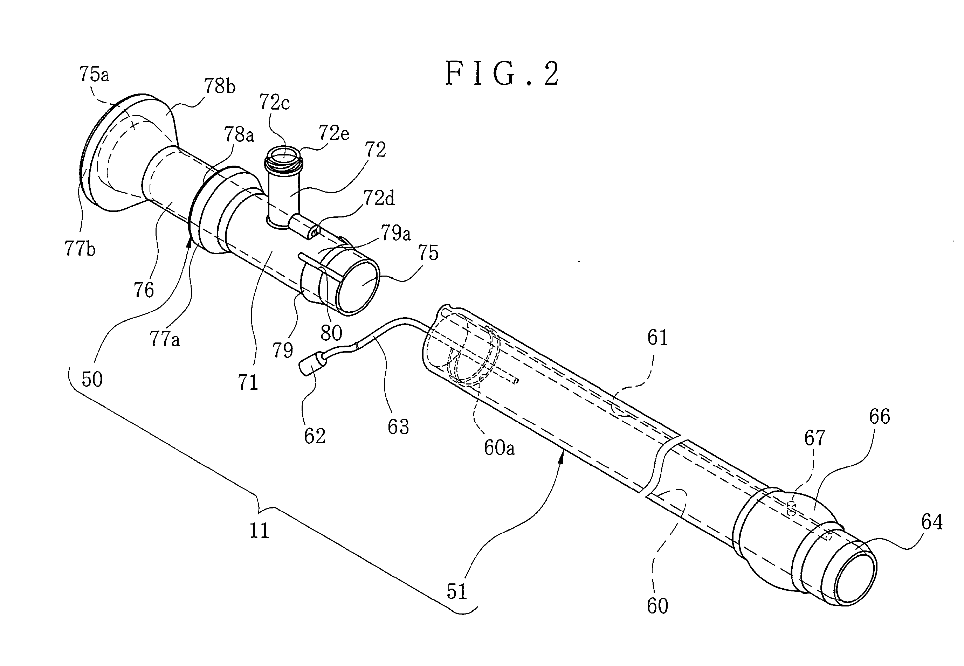

In Fig. 2, a tube lumen 60 and an inflation lumen 61 or fluid channel are formed through the elongated tube 51 axially. The tube lumen 60 receives entry of the insertion tube 13 of the endoscope 10. As viewed in a cross section of the elongated tube 51, the tube lumen 60 is circular. An inner diameter of the tube lumen 60 is slightly greater than an outer diameter of the insertion tube 13. The inner surface of the tube lumen 60 is coated with hydrophilic coating agent or lubricant coating agent, for example, polyvinyl pyrrolidone.

To use the overtube apparatus 11, lubricating fluid such as water is supplied to the inner surface of the tube lumen 60 or clearance space between the insertion tube 13 and the elongated tube 51, to reduce friction between the insertion tube 13 and the elongated tube 51. There is a tube connector 62. A syringe (not shown) is used to inject the lubricating fluid through the tube connector 62. A flow tube 63 of a small bore has a proximal end connected to the tube connector 62, and has a distal end connected to a proximal end of the tube lumen 60. The lubricating fluid injected in the tube connector 62 is supplied to the inner surface of the tube lumen 60 through the flow tube 63.

An inclined wall 64 is formed at a distal end of the elongated tube 51 for decreasing the inner diameter. When the insertion tube 13 of the endoscope 10 is entered in the tube lumen 60, the inclined wall 64 reduces a gap between the insertion tube 13 and the distal end of the elongated tube 51, to prevent leakage of the lubricating fluid in a distal direction of the elongated tube 51.

A distal balloon end is a portion of the balloon 66. An end of the inflation lumen 61 is closed at a fixed point of the distal balloon end. An inflation opening 67 is formed in the outer surface of the elongated tube 51. The inflation lumen 61 extends to communicate with the inflation opening 67. The inflation opening 67 is positioned inside the balloon 66. Air is drawn in and out through the inflation opening 67 to expand and compress the balloon 66. A fluid channel 72 is formed with the handle device 50. A proximal end of the inflation lumen 61 is connected to the fluid channel 72 to be described later.

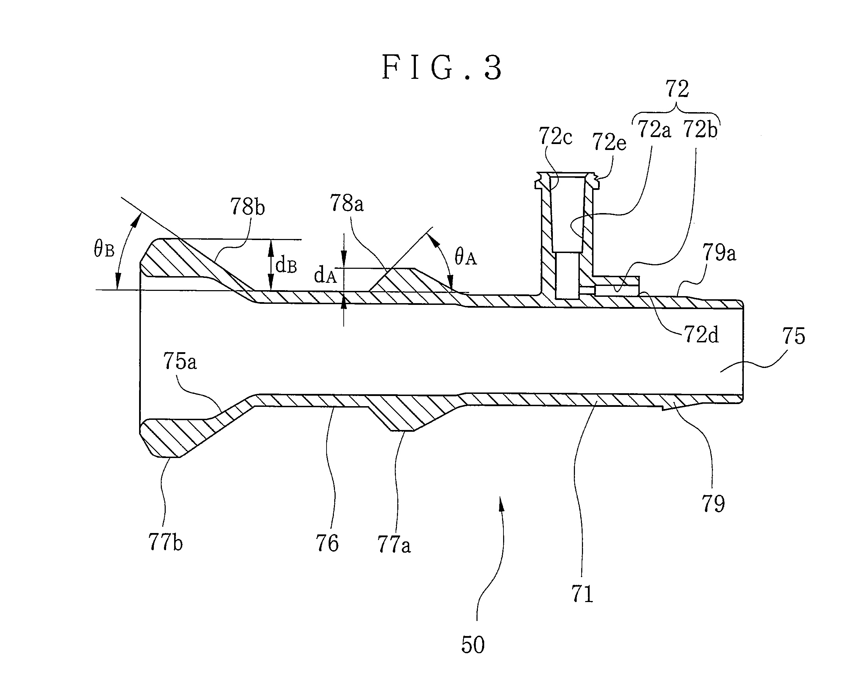

In Figs. 2 and 3, a handle sleeve 71 is a base portion of the handle device 50, and combined with an end tube portion having the fluid channel 72. The handle sleeve 71 is connected to the proximal end of the elongated tube 51. The end tube portion projects from a peripheral surface of the handle sleeve 71.

A handle lumen 75 with an inner surface is formed through the handle sleeve 71, and communicates with the tube lumen 60 when a distal end of the handle sleeve 71 is fitted in the tube lumen 60. An inclined inner surface 75a of a proximal opening is formed in a proximal end of the handle lumen 75, and has an inner diameter increasing gradually in a proximal direction. The inclined inner surface 75a guides the insertion tube 13 and facilitates its entry into the tube lumen 60.

The handle sleeve 71 includes a small diameter portion 76, large diameter portions 77a and 77b, and inclined portions 78a and 78b. The large diameter portions 77a and 77b have larger diameters than the small diameter portion 76. The large diameter portion 77a is disposed at a distal end of the small diameter portion 76. The large diameter portion 77b is disposed at a proximal end of the small diameter portion 76. The inclined portions 78a and 78b extend smoothly to connect the small diameter portion 76 to the large diameter portions 77a and 77b. Each diameter of the inclined portions 78a and 78b increases gradually toward the large diameter portions 77a and 77b.

In Fig. 3, let dA and dB be radial differences or step sizes of the large diameter portions 77a and 77b with reference to the small diameter portion 76. Let θA and θB be rising angles of the inclined portions 78a and 78b with reference to the small diameter portion 76. If values of the radial differences dA and dB and the rising angles θA and θB are high, prevention of slip in handling the handle device 50 will be more effective. Should the radial differences dA and dB and the rising angles θA and θB be too high, load to fingers handling the handle device 50 will be excessively high. Thus, it is preferable in the invention to determine the radial differences dA and dB equal to or more than 3.5 mm and equal to or less than 8 mm, and the rising angles θA and θB equal to or more than 30 degrees and equal to or less than 80 degrees after considering effects against slip in handling the handle device 50 and load to fingers of the operator.

Materials for the handle device 50 are not limited to ABS resin but can be such having sufficient rigidity for manual holding and without use of silicone resin. Specifically, materials for the handle device 50 can have a Shore A hardness equal to or more than 90, namely ABS resin, polypropylene (PP), polyethylene (PE), polyvinyl chloride (PVC) and the like. The Shore A hardness is generally used as an index for plastic materials. Its definition and measuring method are described in the Japanese Industrial Standard (JIS) K7215.

A projection 79 of a C shape is formed at a distal end of the handle sleeve 71. An engageable groove 60a is formed in the elongated tube 51 near to the proximal end of the tube lumen 60 in Fig. 2, and is engaged with the projection 79. The engagement of the projection 79 prevents lubricating fluid from leaking out of the distal end of the handle device 50 and the proximal end of the tube lumen 60. Note that a portion of the engageable groove 60a is closed at one peripheral section which is short of a position of the inflation lumen 61. A peripheral surface 79a is defined by partially removing the projection 79 at a point in the circumferential direction.

A groove 80 of Fig. 2 is formed in a tube wall of the handle sleeve 71 at its distal end to extend transversely with the peripheral surface 79a. The distal end of the handle sleeve 71 is entered in the tube lumen 60 of the elongated tube 51 to engage the projection 79 with the engageable groove 60a. Then the flow tube 63 in the tube lumen 60 extends through the groove 80 and becomes protruded from the distal end of the elongated tube 51.

The end tube portion having the fluid channel 72 is in an L shape and is a combination of a first conduit 72a and a second conduit 72b. The first conduit 72a extends in a radial direction of the handle sleeve 71. The second conduit 72b extends in the longitudinal axis direction of the handle sleeve 71 from one end of the first conduit 72a. A first fluid opening 72c is defined at a proximal end of the first conduit 72a. A second fluid opening 72d is defined at a distal end of the second conduit 72b. A male thread 72e is formed on a wall around the first fluid opening 72c. The male thread 72e operates for connection.

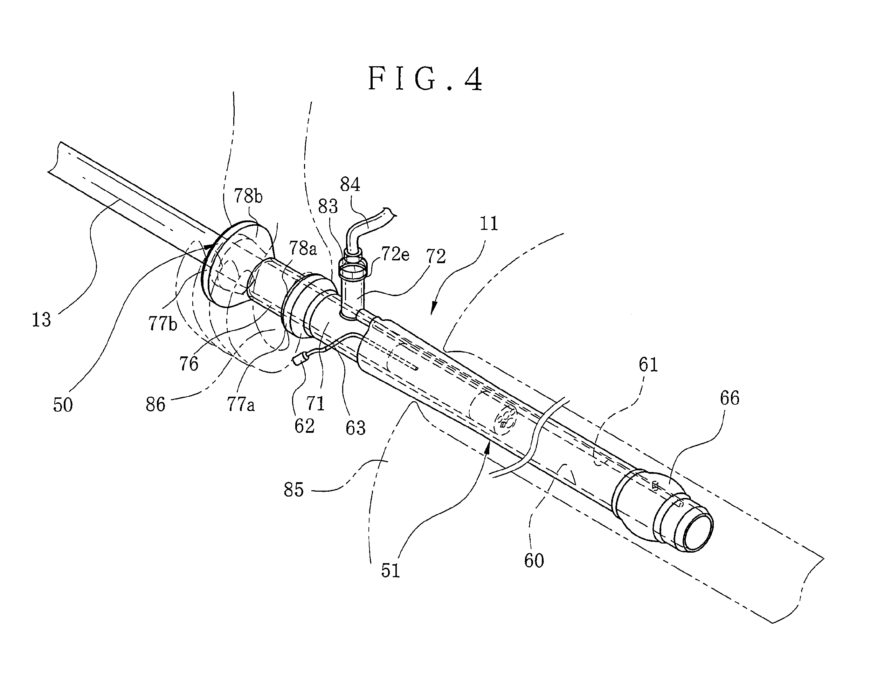

In Figs. 2 and 4, the second fluid opening 72d of the fluid channel 72 communicates with the proximal end of the inflation lumen 61 when a distal end of the handle sleeve 71 is fitted in the tube lumen 60. A tube connector 83 is connected to the male thread 72e. There is a balloon controller 82 of Fig. 1. A flow tube 84 is connected between the tube connector 83 and the balloon controller 82. A female thread is formed inside the tube connector 83. The male thread 72e is helically engaged with the female thread to prevent leakage of fluid. The balloon controller 82 supplies or draws air to expand or compress the balloon 66.

In Fig. 1, the balloon controller 82 causes air or other fluid to flow into and out of the balloon 66. A control panel or switch device 100 and a balloon monitor display panel 101 are associated with the balloon controller 82. A front surface of the balloon controller 82 has a power switch, a stop switch and the like.

The balloon controller 82 supplies air to the balloon 66 for expansion, and also controls pressure of the air to keep the balloon 66 expanded. Also, the balloon controller 82 operates for suction of air from the balloon 66 for compressing, and controls the air pressure at a constant level for keeping the balloon 66 compressed. Various switches are included in the control panel 100 for the purpose of inputting command signals similarly to the balloon controller 82 but manually. A cable electrically connects the control panel 100 to the balloon controller 82.

The balloon monitor display panel 101 displays various data in operation of expanding and compressing the balloon 66, such as a pressure value, states of the expansion and compression. Note that those data on the display panel 35 can be superimposed visibly on an object image of a body part viewed in the endoscope 10.

The operation of manipulating the endoscope system 2 of the above construction is described now. In Fig. 4, the insertion tube 13 of the endoscope 10 is entered in the tube lumen 60 of the overtube apparatus 11. A coating of lubricating jelly is applied to the overtube apparatus 11. The insertion tube 13 and the overtube apparatus 11 are entered into a body cavity in a body 85 alternately in a push method. The balloon 66 is expanded when required, to anchor the overtube apparatus 11 in the body 85. Then the insertion tube 13 is entered further with advance in the body cavity after anchoring the overtube apparatus 11 in the body 85.

To move the overtube apparatus 11 back and forth repeatedly relative to the body 85 or in inward and outward directions, fingers 86 of a doctor or operator grasp the handle device 50 to push and pull the overtube apparatus 11. As described heretofore, the radial differences or step sizes dA and dB are set equal to or more than 3.5 mm and equal to or less than 8 mm, and the rising angles θA and θB are set equal to or more than 30 degrees and equal to or less than 80 degrees, so that it is possible to continue the manipulation without excessive load to the fingers 86. Also, it is possible to produce the handle device 50 at a low cost because the material for the handle device 50 has a Shore A hardness equal to or more than 90. This is typically preferable in construction without use of silicone resin.

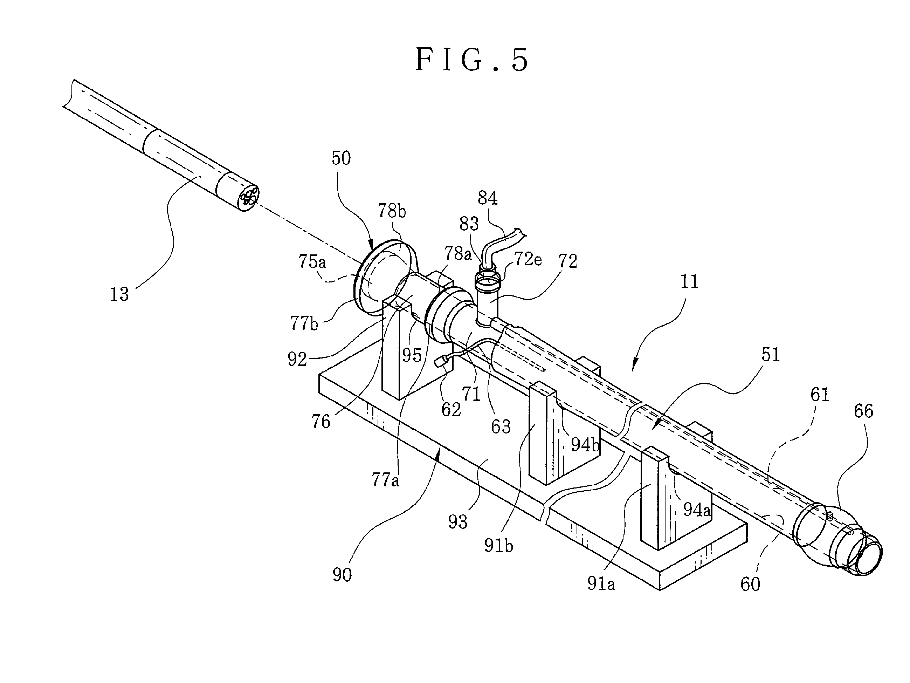

In the above embodiments, the handle device 50 is manually held by a hand of the doctor or operator for use. In Fig. 5, a tube support 90 or stand is illustrated, and used for placement of the overtube apparatus 11. Various modifications of the tube support 90 are possible in the invention. In the embodiment, the tube support 90 includes a base plate 93, support plates 91a and 91b and a support plate 92. The support plates 91a and 91b protrude up from the base plate 93, and support distal and proximal ends of the elongated tube 51. The support plate 92 protrudes up from the base plate 93, and supports the handle device 50. Each of the support plates 91a, 91b and 92 is a plate of a quadrilateral form. Recesses 94a and 94b of a semicircular shape are formed in the support plates 91a and 91b. A recess 95 of a semicircular shape is formed in the support plate 92. A diameter of the recesses 94a and 94b in the support plates 91a and 91b is determined suitably for the outer surface of the elongated tube 51. The elongated tube 51 is received by the recesses 94a and 94b and held by the support plates 91a and 91b.

A thickness of the support plate 92 is smaller than a length of an axis of the small diameter portion 76. The recess 95 in the support plate 92 has an inner diameter equal to or more than an outer diameter of the small diameter portion 76, and less than the outer diameters of the large diameter portions 77a and 77b. The support plate 92 supports the handle device 50 after moving the small diameter portion 76 of the handle device 50 in a downward direction to the recess 95. The handle device 50 on the support plate 92 is regulated in the radial direction by positioning of the small diameter portion 76 in the recess 95, and is prevented from removal in a longitudinal axis direction by contact of the edges of the recess 95 with the inclined portions 78a and 78b. The overtube apparatus 11 held on the tube support 90 constructed above is regulated in the radial direction and the longitudinal axis direction, so that a doctor or operator can manipulate the overtube apparatus 11 reliably for entry.

Forms of using the overtube apparatus are not limited to the above examples. It is possible to use a holder disposed at an end of an arm suspended downwards, and to hold the handle device of the overtube apparatus and the elongated tube. Also, the handle device of the overtube apparatus and the elongated tube can be supported on a belt which can be suspended from a shoulder of a doctor or operator. The small diameter portion 76 is supported in a manner similar to the above embodiment, and can be regulated in a radial direction and a longitudinal axis direction of the handle device 50. Effects similar to the above embodiment can be obtained.

In the above embodiment, the overtube apparatus has the balloon. However, a handle device of the invention can be used for an overtube apparatus without balloon. Of course, the fluid channel 72 can be omitted from a handle device of the invention.

### EXAMPLES

The invention is not limited to the examples in the following description. In Table 1, evaluation of results of experiments of grippability of handle devices were indicated, with differences in the values of the radial differences dA and dB and rising angles θA and θB. For grades of the grippability, o denotes a very good product without slip in manual handling, without load to fingers, and without a problem, and Δ denotes a sufficiently acceptable product but with unacceptable touch in manual handling or with small load to fingers, and x denotes a poor product with slip in manual handling or with load to fingers. In Table 1, only the radial difference dB larger than the radial difference dA was indicated and the radial difference dA was omitted, because the large diameter portion 77b on the proximal side was larger than the large diameter portion 77a on the distal side. Note that the small diameter portion 76 had a diameter of 15 mm, and a length of an axis of the small diameter portion 76 was 10-20 mm.

**Table 1**

| Sample No. | Radial difference (mm) | Rising angle (distal inclination, degrees) | Rising angle (proximal inclination, degrees) | Evaluation of grippability |
|---|---|---|---|---|
| 1 | 2.0 | 45 | 40 | x |
| 2 | 3.5 | 45 | 40 | Δ |
| 3 | 5.0 | 45 | 40 | O |
| 4 | 6.5 | 45 | 40 | O |
| 5 | 8.0 | 45 | 40 | O |
| 6 | 9.5 | 45 | 40 | X |
| 7 | 6.5 | 45 | 20 | X |
| 8 | 6.5 | 45 | 40 | O |
| 9 | 6.5 | 45 | 60 | O |
| 10 | 6.5 | 45 | 80 | Δ |
| 11 | 6.5 | 45 | 90 | X |
| 12 | 6.5 | 20 | 40 | X |
| 13 | 6.5 | 40 | 40 | O |
| 14 | 6.5 | 60 | 40 | O |
| 15 | 6.5 | 80 | 40 | O |
| 16 | 6.5 | 90 | 40 | X |

Evaluation was indicated in Table 1. According to Samples 2-5, 8-10 and 13-15 in which the radial difference dB was equal to or more than 3.5 mm and equal to or less than 8 mm and the rising angles θA and θB were equal to or more than 30 degrees and equal to or less than 80 degrees, the handle device as product was acceptable or usable. According to Samples 1, 6, 7, 11, 12 and 16, grippability of the handle device was low. According to Samples 1, 7 and 12 in which the radial difference dB was less than 3.5 mm or the rising angles θA and θB were less than 30 degrees, slip of the handle device as product was serious because of an excessively small space of touch of fingers. According to Samples 6, 11 and 16 in which the radial difference dB was more than 8 mm or the rising angles θA and θB were more than 80 degrees, load to fingers from the handle device as product was excessively large.

Although the present invention has been fully described by way of the preferred embodiments thereof with reference to the accompanying drawings, various changes and modifications will be apparent to those having skill in this field. Therefore, unless otherwise these changes and modifications depart from the scope of the present invention, they should be construed as included therein.

## Claims

1. A handle device of a barrel shape for use at a proximal end (60a, 63) of an overtube apparatus (11) for receiving entry of an insertion tube (13) of an endoscope (10) to guide said insertion tube into a body cavity, **characterized in** comprising:
a small diameter portion (76);
first and second large diameter portions (77a, 77b), disposed on sides of distal and proximal ends (78a, 78b) of said small diameter portion, having a larger diameter than said small diameter portion, wherein a radial difference (dA, dB) between each of said first and second large diameter portions and said small diameter portion is equal to or more than 3.5 mm and equal to or less than 8 mm;
first and second inclined portions (78a, 78b), arranged to extend from said small diameter portion to respectively said first and second large diameter portions, and inclined at a rising angle (θA, θB) equal to or more than 30 degrees and equal to or less than 80 degrees with reference to said small diameter portion;
wherein said small diameter portion, said first and second large diameter portions, and said first and second inclined portions are formed from a material having a Shore A hardness equal to or more than 90.

2. A handle device as defined in claim 1, **characterized in that** said material is constituted by at least one of ABS resin, polypropylene, polyethylene, and polyvinyl chloride.

3. A handle device as defined in claim 1 or 2, **characterized in** further comprising a fluid channel for supplying air for a balloon positioned at a distal end of said overtube apparatus in communication with a lumen in said overtube apparatus.

4. A handle device as defined in any one of claims 1-3, **characterized in** further comprising:
a handle sleeve having a handle peripheral surface on which said small diameter portion, said first and second large diameter portions and said first and second inclined portions are formed; and
a handle lumen, defined through said handle sleeve, for receiving entry of said insertion tube.

5. A handle device as defined in claim 4, **characterized in that** said overtube apparatus has a proximal end opening of an elongated tube thereof, and a distal end of said handle sleeve is inserted in said proximal end opening for connection.

6. A handle device as defined in claim 4 or 5, **characterized in that** said first large diameter portion has a smaller diameter than a diameter of said second large diameter portion.

7. A handle device as defined in any one of claims 4-6, **characterized in** further comprising a proximal opening, formed to communicate with a proximal end of said handle lumen, and to extend through said second large diameter portion, said proximal opening having an inner diameter larger than an inner diameter of said handle lumen.

8. A handle device as defined in any one of claims 4-7, **characterized in that** said fluid channel includes:
a first conduit, positioned on said handle peripheral surface, for extending crosswise to a longitudinal axis of said handle lumen;
a second conduit for communicating with an end of said first conduit close to said longitudinal axis, and for extending along said longitudinal axis in a distal direction.

9. A handle device as defined in any one of claims 4-8, **characterized in that** said fluid channel is constituted by an end tube portion formed with said handle peripheral surface and on a distal side from said first large diameter portion.

10. An overtube apparatus for receiving entry of an insertion tube (13) of an endoscope (10), **characterized in** comprising:
(A) an elongated tube (51) for entry in a body cavity, said elongated tube having a tube lumen (60) for receiving said insertion tube;
(B) a handle device of a barrel shape connected to a proximal end (60a, 63) of said elongated tube;
said handle device including:
a small diameter portion (76);
first and second large diameter portions (77a, 77b), disposed on sides of distal and proximal ends (78a, 78b) of said small diameter portion, having a larger diameter than said small diameter portion, wherein a radial difference (dA, dB) between each of said first and second large diameter portions and said small diameter portion is equal to or more than 3.5 mm and equal to or less than 8 mm;
first and second inclined portions (78a, 78b), arranged to extend from said small diameter portion to respectively said first and second large diameter portions, and inclined at a rising angle (θA, θB) equal to or more than 30 degrees and equal to or less than 80 degrees with reference to said small diameter portion;
wherein said small diameter portion, said first and second large diameter portions, and said first and second inclined portions are formed from a material having a Shore A hardness equal to or more than 90.
